# EUROPEAN PATENT APPLICATION

(11) **EP 2 359 880 A2**
(43) Date of publication of application: **24.08.2011**
(21) Application number: 10190856.4
(22) Date of filing: 04.02.2009
(51) Int. Cl.: A61M 5/142, G06F 19/00, A61M 5/172

(54) **Drug delivery system with wireless monitor**

(30) Priority: 04.02.2008 US 25859 P
(62) Divisional of application: 09707503.0
(71) Applicant: Nilimedix Ltd., 31905 Haifa (IL)
(72) Inventor: Shekalim, Avraham, 36842, Nesher (IL)
(74) Representative: Modiano, Micaela Nadia

(57) **Abstract**

A portable drug delivery system (10) includes a remote unit (14) which serves as a wireless monitor for a drug delivery device (12). Programming of the drug delivery device (12) can be achieved by use of the remote unit (14), but requires tactile intervention by the user with the drug delivery device, thereby avoiding inadvertent or unauthorized changes to the drug delivery settings via the wireless unit. Also disclosed are configurations for receiving and retaining a drug cartridge (42) within a disposable unit (40) of the drug delivery device, and a kit providing user- selectable form factor for the infusion set used with the drug delivery device, allowing the user to select either an integrated infusion set (112) or an external infusion (106) set for each session of use.

## Description

### FIELD AND BACKGROUND OF THE INVENTION

The present invention is a drug delivery system with a wireless monitor, and/or which provides user selectable infusion set configurations and/or which facilitates use of pre-filled or user fillable drug containers of varying dimensions.

There exist many types of portable drug delivery devices which are either attached directly to the body of the user or are carried in a pocket and delivery a drug via an infusion set. A particularly significant subset of such devices are insulin pumps used by individuals suffering from diabetes to control blood sugar levels.

In many cases, the drug delivery device, which will be referred to herein colloquially as a "pump", is located under clothing and/or may be in a location on the body which is either physically awkward or socially awkward to access. For this reason, various products have been developed which employ a wireless remote control unit with a suitable display and controls through which the user can control operation of the pump, for example, setting different basal profiles for the underlying rate of drug delivery and actuating short term "bolus" delivery for supplementing the underlying basal rate.

The use of a remote control has various advantages as stated, but also presents certain potential dangers. Specifically, since the operation of the pump is controlled via the remote controller, there exists a risk of inadvertent operation of the device by the user himself, or that an unauthorized person may play with the controller and modify the operation of the pump in an undesirable or even dangerous manner. This possibility is particularly of concern for children who often play with any gadget which falls into their hands in order to try to figure out how it works.

Regarding the form factor of drug delivery devices, it is notable that commercial portable drug delivery devices may be subdivided into two main groups based on the positioning of the infusion interface. A first type has a built-in needle mounted on the body of the device. In this case the device is affixed directly to the skin of the user, and drug delivery occurs within the footprint of the device itself, or immediately adjacent thereto. A second type of drug delivery device delivers the drug to an external infusion set via a flexible tube. Each of these types of pump has a corresponding set of advantages and disadvantages which the user must weigh carefully before deciding which type of pump to purchase. In many cases, the choice between these options may be a primary consideration in a choice of pump, since the user is subsequently limited to the corresponding mode of use, either needing to deal with sometimes inconvenient or unsightly lengths of flexible tubing, or alternatively needing to keep the entire pump directly attached to the skin at all times during use.

Regarding the storage and supply of a drug for commercial portable drug delivery devices, here too, it will be noted that different drug delivery devices commercially available may also be classified according to whether they use a user-fillable, drug reservoir/cartridge or a prefilled drug reservoir/cartridge. Many devices employ a user-fillable cartridge in which case the cartridge is supplied empty and the user employs a needle and syringe to draw a quantity of drug from a container, which may be refrigerated, and inject it into the cartridge. This approach provides flexibility as to the quantity of drug which is loaded into the cartridge, but is accompanied by several drawbacks: a risk of introducing air bubbles into the system which may interfere with reliable dosing of the drug, a risk of contamination of the drug and/or cartridge during filling, and a dislike many users have for needing to handle needles during the filling process.

As an alternative, certain manufacturers provide a prefilled cartridge which already contains a quantity of drug suitable for most users over the operative lifetime of the cartridge. For example, in the case of insulin where the cartridge is designed to be used for a maximum of three days, a standard quantity of 3 cc. of insulin is typically provided per cartridge. Any amount unused at the end of the three day period is discarded. This approach has advantages of convenience, sterility and avoids introducing air bubbles. The provision of a standard quantity, however is wasteful and expensive for users whose dosage requirements are significantly less than the standard 3 cc. in three days.

Existing portable drug delivery devices are also typically highly specific in the dimensions and design of the drug cartridge which can be used, leaving the user dependent upon particular suppliers. Even a relatively small difference in length or diameter of a cartridge renders it non-interchangeable between the various different products on the market.

There is therefore a need for a portable drug delivery device which would provide the convenience of controlling the device and verifying that it is operating properly for a range of normal operations (e.g., execution of a pre-set basal profile and delivery of additional bolus doses on demand) without requiring the body-mounted pump to be physically uncovered and viewed, but which would at the same time prevent inadvertent or unauthorized changes to the drug delivery program via a remote controller. It would also be advantageous to provide a portable drug delivery system which would allow the user to select either a built-in or external infusion set configuration. Finally, it would also be advantageous to provide a portable drug delivery device which can employ interchangeably prefilled containers of varying dimensions and/or user fillable containers.

### SUMMARY OF THE INVENTION

The present invention is a drug delivery system with a wireless monitor, and/or which provides user selectable infusion set configurations and/or which facilitates use of pre-filled or user fillable drug containers of varying dimensions.

According to the teachings of the present invention there is provided, a drug delivery system for delivering a drug to the body of a user, the system comprising: (a) a drug delivery device including: (i) a housing; (ii) a reservoir containing a quantity of the drug, the reservoir being located at least partially within the housing; (iii) an infusion arrangement for delivering the drug into the body of the user; (iv) a flow controller for controlling a flow of the drug from the reservoir to the infusion arrangement; and (v) a first communication device associated with the flow controller and including at least a transmitter, the first communication device transmitting wirelessly information indicative of a current state of operation of the flow controller; and (b) a remote unit including: (i) a display; (ii) a plurality of user input controls; (iii) a remote unit controller associated with the display and the user input controls; and (iv) a second communication device associated with the remote unit controller and including at least a receiver, the second communication device receiving the wirelessly transmitted information and supplying the information to the remote unit controller for display on the display, wherein the remote unit is configured to receive user input via the user input controls to define a required change in operation of the flow controller, and wherein the drug delivery device is configured to require tactile intervention by the user prior to the required change in operation being implemented.

According to a further feature of the present invention, the first communication device includes a receiver and the second communication device includes a transmitter, and wherein data indicative of the required change in operation is transmitted wirelessly from the remote unit to the drug delivery device, the drug delivery device further comprising a manually operable user input, the tactile intervention requiring actuation of the manually operable user input to confirm the required change in operation.

According to a further feature of the present invention, the manually operable user input is implemented as a readily accessible button configured to be operable by pressure of a finger applied through a garment.

According to a further feature of the present invention, there is also provided a tethered connection selectively deployable to provide a tethered link between the remote unit and the drug delivery device, wherein the tactile intervention requiring deployment of the tethered connection.

According to a further feature of the present invention, the tethered connection is a wired communication link along which is transmitted data indicative of the required change in operation.

According to a further feature of the present invention, there is also provided a data storage device, wherein each of the drug delivery device and the remote unit comprises a connector for removable attachment of the data storage device, and wherein the tactile intervention requires transfer of the data storage device from the remote unit to the drug delivery device.

There is also provided according to the teachings of the present invention, a method for operating a drug delivery system for delivering a drug to the body of a user, the method comprising the steps of: (a) providing a drug delivery device including a reservoir containing a quantity of the drug and a flow controller for controlling a flow of the drug from the reservoir to the body of the user; (b) providing a remote unit including a display and a plurality of user input controls; (c) deploying the drug delivery device to deliver the drug to the body of the user in a first defined manner; (d) during operation of the drug delivery device, transmitting wirelessly from the drug delivery device to the remote unit information indicative of a current state of operation of the drug delivery device for display on the display; (e) inputting to the remote unit via the user input controls a required change in operation of the drug delivery device; (f) transferring to the drug delivery device data indicative of the required change in operation; and (g) changing the operation of the drug delivery device in accordance with the data, wherein at least one of the transferring and the changing requires tactile intervention by the user with the drug delivery device.

According to a further feature of the present invention, the tactile intervention comprises manual operation of an input device located on the drug delivery device.

According to a further feature of the present invention, the user input on the drug delivery device is implemented as a readily accessible button configured to be operable by pressure of a finger applied through a garment.

According to a further feature of the present invention, the tactile intervention comprises deployment of a tethered connection between the remote unit and the drug delivery device.

According to a further feature of the present invention, the tethered connection is a wired communication link along which is transmitted data indicative of the required change in operation.

According to as further feature of the present invention, the tactile intervention comprises transfer of a data storage device from the remote unit to the drug delivery device.

There is also provided according to the teachings of the present invention, a drug delivery device for delivering a drug to the body of a user, the device comprising a disposable unit for receiving a cartridge containing a drug to be delivered, the disposable unit comprising: (a) a channel for receiving the cartridge containing the drug to be delivered, the channel having an opening and a direction of extension; (b) a loading spring deployed within the channel for applying a loading force on the cartridge when inserted within the channel, the loading force acting along the direction of extension towards the opening; and (c) a retention configuration deployed near the opening, the retention configuration including a plurality of rearwardly directed resilient teeth configured: (i) to retain the loading spring in a pre-compressed state within the channel prior to insertion of the cartridge, (ii) to flex outwards to allow insertion of the cartridge between the teeth, and (iii) to assume a retaining position engaging a recessed portion of the cartridge when the cartridge is fully inserted.

According to a further feature of the present invention, the plurality of rearward directed resilient teeth are formed as part of a collar deployed within the opening.

According to a further feature of the present invention, there is also provided a user-fillable cartridge sized and shaped for insertion into the channel.

According to a further feature of the present invention, the cartridge has an open base and a displaceable piston, and wherein the loading spring is configured to apply a load on the displaceable piston so as to pressurize the contents of the cartridge.

There is also provided according to the teachings of the present invention, a modular drug delivery system kit for delivering a drug to the body of a user, the kit comprising: (a) a drug delivery device comprising a disposable unit having an outlet arrangement with at least one outlet for release of a drug for infusion into the body of the user; (b) a first infusion set including a canula in fluid connection with a length of flexible tube for delivery of the drug into the body of the user at a location spaced from the drug delivery device, the first infusion set being configured for fluid connection to the outlet arrangement; and (c) a second infusion set including a canula deployable in fixed relation to a body contact element, the body contact element being configured to cooperate with the drug delivery device to define a fixed spatial alignment between the body contact element and the drug delivery device, and to define a fluid flow path from the outlet arrangement to the canula, wherein one of the first infusion set and the second infusion set is attached to the drug delivery device to provide a user selected form factor for the drug delivery system.

According to a further feature of the present invention, the outlet arrangement includes a first outlet for attachment of the first infusion set and a second outlet for attachment of the second infusion set.

According to a further feature of the present invention, the at least one outlet is formed with a septum needle recessed within a socket, and with an elastomer seal deployed within the socket so as to seal the end of the septum needle prior to attachment of the first or second infusion set.

### BRIEF DESCRIPTION OF THE DRAWINGS

The invention is herein described, by way of example only, with reference to the accompanying drawings, wherein:
FIG. 1 is a schematic block diagram of a drug delivery system employing a drug delivery device and a wireless monitor, constructed and operative according to the teachings of the present invention;
FIGS. 2A-2C are schematic representations of the drug delivery system of FIG. 1, illustrating three options for implementing programming of the drug delivery device via the wireless monitor in a manner requiring tactile intervention by the user;
FIGS. 3A and 3B are isometric views of a non-limiting exemplary implementation of the drug delivery device and the wireless monitor of the system of FIG. 1, respectively;
Fig. 4 is an isometric view of a disposable unit from the drug delivery device of FIG. 3A with a drug container ready for insertion;
FIGS. 5A-5C are schematic partially-cut-away views of the drug delivery device of FIG. 3A prior to, during and after insertion of the drug container, respectively;
FIGS. 6A-6C are isometric, axial and side views, respectively, of a retention configuration from the drug delivery device of FIGS. 5A-5C;
FIG. 6D is a cross-sectional view taken along the line A-A in FIG. 6B;
FIG. 7 is an isometric view of a drug delivery system kit including the drug delivery device of FIG. 3A and two infusion sets providing alternative form factors for attachment to the drug delivery device;
FIGS. 8A and 8B are cross-sectional views through an outlet port of the drug delivery device of FIG. 7 taken prior to and subsequent to attachment of the external infusion set, respectively;
FIGS. 9A and 9B are isometric views of the drug delivery device of FIG. 3A prior to and subsequent to attachment of an integrated infusion set, respectively; and
FIGS. 10A and 10B are cross-sectional views taken parallel to an infusion canula of FIGS. 9A and 9B, respectively.

### DESCRIPTION OF THE PREFERRED EMBODIMENTS

The present invention is a drug delivery system with a wireless monitor, and/or which provides user selectable infusion set configurations and/or which facilitates use of pre-filled or user fillable drug containers of varying dimensions.

The principles and operation of drug delivery systems and corresponding methods according to the present invention may be better understood with reference to the drawings and the accompanying description.

### Overview

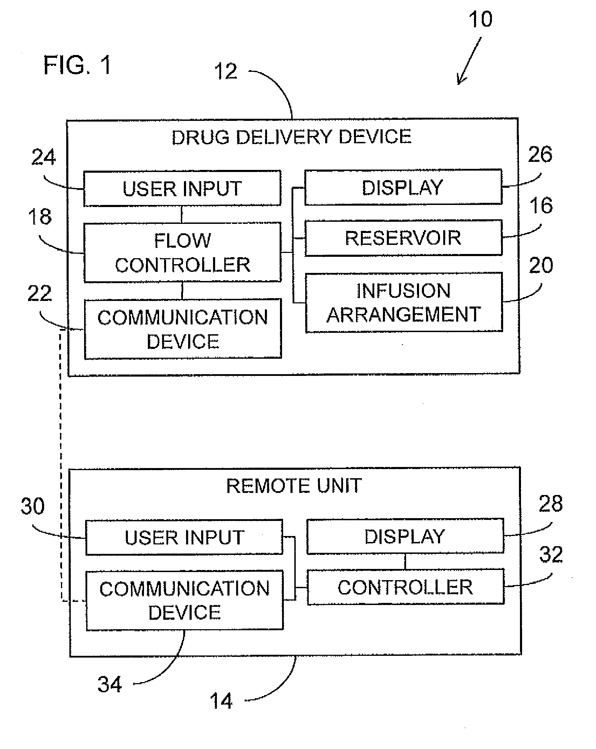

By way of introduction, the present invention relates to a number of sets of features which may each be used independently to advantage, but which will be exemplified herein primarily by reference to a particularly preferred embodiment which combines the various features. Specifically, a first aspect of the present invention relates to provision of remote unit which serves as a wireless monitor for a drug delivery device wherein programming of the drug delivery device can be achieved by use of the remote unit but requires tactile intervention by the user with the drug delivery device, thereby avoiding inadvertent or unauthorized changes to the drug delivery settings via the wireless unit. This aspect of the invention will be described below primarily with reference to FIGS. 1-3B.

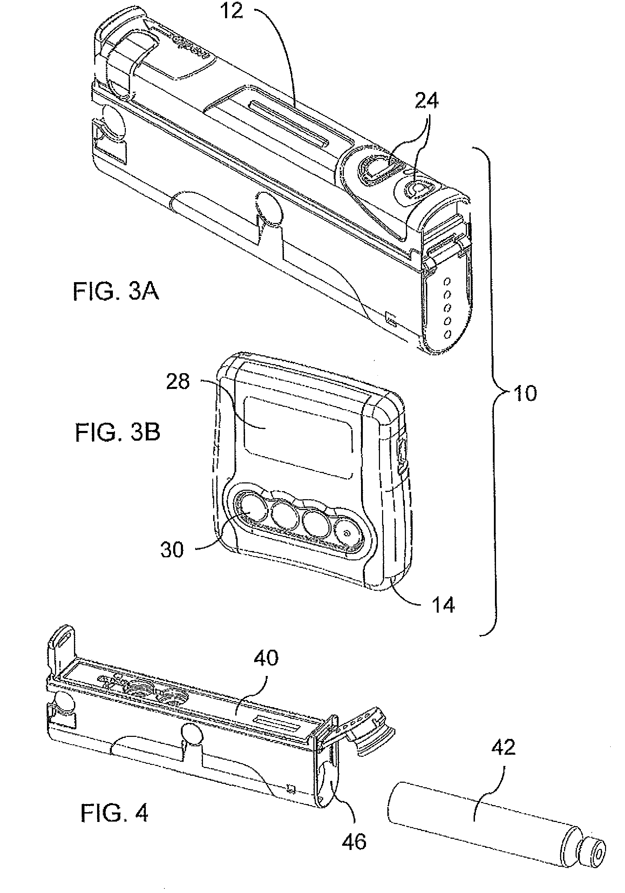

A second aspect of the present invention relates to a configuration for receiving and retaining a drug container within a disposable unit of the drug delivery device. This aspect of the invention will be described below primarily with reference to FIGS. 4-6D.

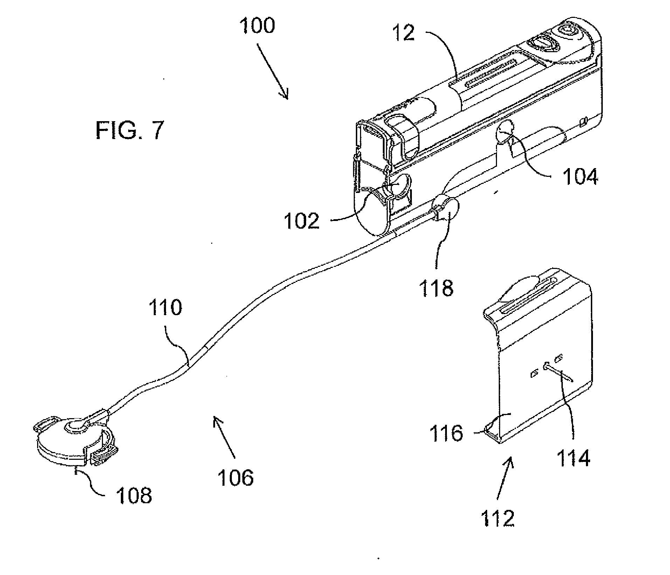

Finally, a third aspect of the present invention relates to a user-selectable form factor for the infusion set used with the drug delivery device, allowing the user to select either an integrated infusion set or an external infusion set for each session of use. This aspect of the present invention will be described below primarily with reference to FIGS. 7-10B.

It should be noted that, unless otherwise noted, the various aspects of the present invention are applicable to a wide range of existing drug delivery devices including, but not limited to, devices with active pumping of a drug from a reservoir to the body and devices with pressurized reservoirs and arrangements of flow-restricting valves. The structure and operation of the flow controller of the pump are therefore not discussed here in detail. By way of one non-limiting but particularly preferred example, the present invention may be implemented using technology described in one or more of the following publications to the assignee of this application: US Patents Nos. 6,736,796; 7,291,126; 7,377,907; 7,311,693; and US Patent Application Publication No. 2007/0250007 A1.

### Remote Unit with Tactile Intervention

To address the need for convenience of operation of a body-mounted or worn drug delivery device on one hand while avoiding the risks of unauthorized modification of drug delivery rates on the other, one aspect of the present invention provides a system and corresponding mode of operation (and method) in which a remote unit separate from the drug delivery device itself acts as a wireless monitor, but requires some tactile intervention by the user with the drug delivery device before a change will occur in the drug delivery programming.

The primary components according to a typical implementation of this aspect of the present invention are shown schematically in FIG. 1. Specifically, FIGS. 1, 3A and 3B show a drug delivery system, generally designated **10,** constructed and operative according to the teachings of the present invention, for delivering a drug into the body of a user. Generally speaking, drug delivery system **10** includes a drug delivery device **12** and a remote unit **14.** Referring primarily to FIG. 1, drug delivery device **12** has a housing, represented here schematically as the outline of device **12.** A reservoir **16** containing a quantity of the drug to be dispensed is located at least partially within the housing. A flow controller **18** for configured for controlling a flow of the drug from reservoir **16** to an infusion arrangement **20,** which delivers the drug into the body of the user. A first communication device **22,** associated with flow controller **18,** includes at least a transmitter. Typically, although not necessarily, drug delivery device **12** also includes one or more user input **24,** such as buttons, switches and/or knobs, and a display **26.**

Remote unit **14** generally includes a display **28** and a plurality of user input controls **30,** interconnected with a remote unit controller **32.** A second communication device **34,** associated with controller **32** includes at least a receiver.

During normal operation of drug delivery device **12,** first communication device **22** transmits wirelessly information indicative of a current state of operation of flow controller **18.** This information is received by second communication device **34** and supplied to remote unit controller **32** for display on display **28,** thereby providing convenient monitoring of the functioning of the drug delivery device.

According to a particularly preferred feature of this aspect of the present invention, remote unit **14** is configured to receive user input via user input controls **30** to define a required change in operation of flow controller **18.** At the same time, drug delivery device **12** is configured to require tactile intervention by the user prior to implementation of the required change.

The term "tactile intervention" is used herein in the description and claims to refer to any and all actions which include either direct physical contact with the drug delivery device or application of a contact force to some part of the device even through a garment. The force or contact may be implemented directly by the hand of a user or indirectly via some intermediate object. The requirement for tactile intervention with the drug delivery device ensures that no change to the operation of the drug delivery occurs based solely on operations performed at the remote unit, but rather as a result of some action which is intuitively perceived by the user as a direct interaction with the drug delivery device itself. This substantially eliminates the risk of unauthorized or inadvertent tampering with the drug delivery regime via the remote unit.

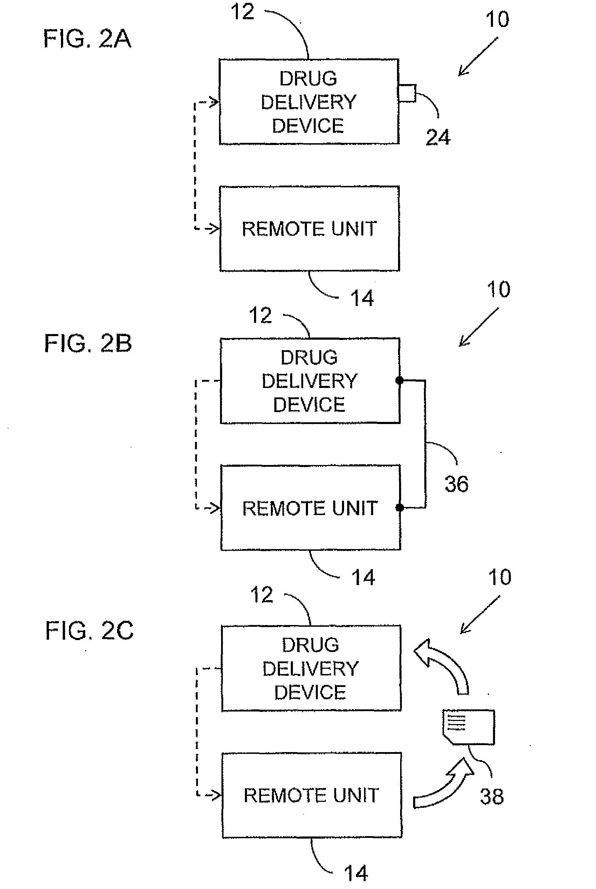

It will be appreciated that the requirement for tactile intervention may be implemented in many ways to achieve the required result. In certain cases, the tactile intervention is required to enable the transfer to the drug delivery device of data indicative of the required change in operation. In other cases, the system may perform data transfer prior to the tactile intervention, instead requiring intervention as a condition for implementing the changes. A few non-limiting examples will now be illustrated with reference to FIGS. 2A-2C.

Referring first to FIG. 2A, this shows an implementation in which both communication devices **22** and **34** include a transmitter and receiver (also referred to as a "transceiver"), allowing bidirectional wireless communication between drug delivery device **12** and remote unit **14.** In this case, data indicative of the required change in operation is preferably transmitted wirelessly from remote unit **14** to drug delivery device **12.** Drug delivery device is preferably configured to require tactile intervention by user actuation of manually operable user input **24** to confirm the required change in operation. For this implementation, user input **24** is preferably implemented as a readily accessible button configured to be discernable and operable by pressure of a finger applied through a garment. This allows the user to manually confirm reprogramming of the drug delivery device performed via the remote unit without uncovering the drug delivery device itself, which may be hidden under clothing or otherwise inconvenient or embarrassing to reveal. Confirmation that the required change in operation has been implemented can then be provided by manual or tactile feedback from the drug delivery device itself and/or via the display or other sensory output from remote unit **14.**

A second non-limiting example is illustrated in FIG. 2B. In this case, a tethered connection **36** between remote unit **14** and drug delivery device **12** is selectively deployed to provide a tethered link between them. Optionally, the tethered connection may be a simple string carrying a mechanical or electronic key or other engagement device (not shown) which engages drug delivery device **12** to provide authorization for implementing the required change to the drug delivery program. In this case, the functionality is essentially equivalent to that of FIG. 2A.

According to an alternative preferred option, tethered connection **36** is a wired communication link, such as a USB cable or the like. In this case, the data indicative of the required change in operation of the drug delivery device may be transmitted via connection **36.** This implementation may optionally employ a unidirectional wireless link from drug delivery device **12** to remote unit **14.**

According to a third non-limiting example illustrated in FIG. 2C, a data storage device **38** is provided for transferring data from remote unit **14** to drug delivery device **12.** Data storage device **38** may be any standard or dedicated form of memory card, memory stick or the like, and drug delivery device **12** and remote unit **14** are implemented with corresponding connectors for removable attachment of data storage device **38.** In this case, the data storage device is first connected to remote unit **14** to receive the new drug delivery program or changes to the existing program. The tactile intervention then requires transfer of data storage device **38** from remote unit **14** to drug delivery device **12** in order to transfer the data indicative of the required change in operation of the drug delivery device. Drug delivery device **12** is preferably configured to execute the updated drug delivery schedule from the connected memory device. Most preferably, the drug delivery device automatically downloads the updated program or changes into an internal memory, thus allowing removal of the plug-in memory device. Confirmation of the updated schedule is transmitted to the remote monitor as before.

In all of the above cases, it should be noted that the changes in operation may be any changes typically required by users of portable drug delivery devices including, but not limited to, changes to a basal drug delivery rate, changes to a basal drug delivery profile, switching between a number of stored basal drug delivery schedules, immediate delivery or scheduling of a bolus dose of drug delivery, and setting parameters for a bolus dose of drug delivery.

Furthermore, although drug delivery system **10** allows remote unit **14** to be used to input required changes to the operation of drug delivery device **12,** this is not necessarily, or even typically, to the exclusion of direct control of drug delivery device **12** via user inputs **24.** In particular, in some cases, certain modifications to operation of the drug delivery device, and in particular, selection and actuation of bolus doses of various sizes, can advantageously be performed using manual input to drug delivery device **12,** but with visual feedback and confirmation supplied via remote unit **14.** In this case, the user selects a desired bolus dose by pressing a button of user inputs **24** on the drug delivery device. This can be done by inserting the user's hand under clothing, or by applying pressure through clothing, without uncovering the drug delivery device. For example, each single press of a finger on the "bolus" button of the device may increment the selected bolus dose by half a unit, while a single long depression of the button, for example of 1.5 second duration, confirms the command to begin delivering the selected bolus. According to a preferred implementation of this aspect of the present invention, after each press on the bolus button of drug delivery device **12,** the drug delivery device transmits a signal indicative of the effect of the button press, e.g., the bolus quantity currently selected, or confirmation that the command has been confirmed and is being delivered. This information is received and then displayed or otherwise reported to the user by remote unit **14.** Thus, the monitoring unit effectively acts as a removable display, indicating in real time what it happening at the drug delivery device in a manner similar to a built in display, but without requiring the user to uncover the drug delivery device itself. As before, since modification of the dosage is achieved through tactile intervention with the drug delivery device, there is no risk to the user of unauthorized or unintended modification of the delivery regime via the remote unit.

At this stage, operation of drug delivery system, corresponding to a method according to this aspect of the present invention, will already be clear. Specifically, drug delivery device **12** is deployed and actuated to deliver the drug to the body of the user in a first defined manner. During operation of the drug delivery device, drug delivery device **12** transmits wirelessly to remote unit **14** information indicative of a current state of operation of drug delivery device **12** for display on display **28.** When a change in operation of drug delivery device **12** is required, details of the required change are input to remote unit **14** via user input controls **30**. In order to implement the change, data indicative of the required change in operation is transferred to the drug delivery device and operation of the drug delivery device is changed in accordance with the data. Either the transfer of the data or the implementation of the change, or both, are contingent on tactile intervention by the user with the drug delivery device.

### Drug Container Retention Configuration

As mentioned above, existing portable drug delivery devices tend to be highly specific as to the design of the container used as the reservoir for storing and dispensing a drug to be delivered. A number of commercially available drug delivery devices employ bottle-like cartridges with a cylindrical storage volume within which a piston slides. The dispensing end of the container typically has a recessed portion, corresponding to a restriction which limits the motion of the piston, followed by a top portion with a piercable elastomeric seal. These cartridges may be variously referred to as bottles, cartridges, or vials. Despite the apparent similarity between the various cartridges of this type supplied with different commercially available devices, most of the devices are based upon precise positive displacement mechanisms which act on the piston directly, and which are highly sensitive to variations in dimensions of the cartridge. For this reason, conventional drug delivery devices typically only operate with the exact model of cartridge for which they were designed, and are not interchangeable.

A further aspect of the present invention relates to a retention configuration for drug containers which provides increased flexibility regarding the dimensions of the container used. This aspect of the invention relates specifically to devices in which the piston of the container is to be springloaded to provide a pressurized reservoir. For simplicity of the structure, it is preferable to allow insertion of the drug container into the device lengthways, i.e., along the direction of the loading spring. However, this presents various practical complications.

Firstly, the diameter of the loading spring must be smaller than that of the drug container in order for the spring to move freely within the inner bore of the container to act on the piston. Secondly, in order to maintain effective spring loading through to the end of the range of motion of the piston, it is necessary to preload the loading spring so that it is retained within the container channel under compression. To prevent the spring from projecting from the channel, a retention configuration is required. However, while retaining the smaller diameter spring, the retention configuration must not obstruct insertion of the larger diameter drug container into the channel.

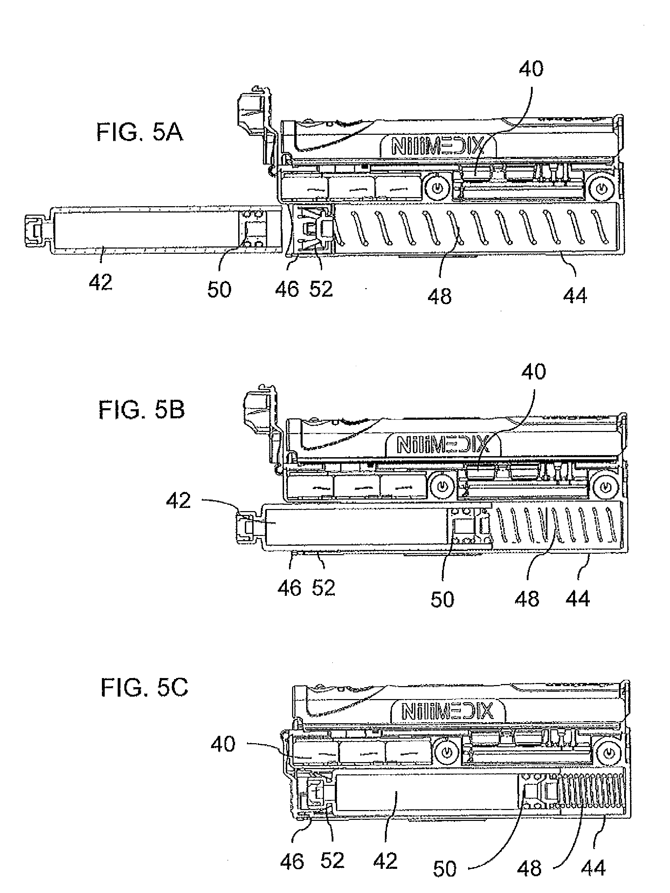

Turning now to FIGS. 4-6D, there is shown a disposable unit **40** from drug delivery device **12** for receiving a cartridge **42** containing a drug to be delivered. As best seen in FIGS. 5A-5C, disposable unit **40** has a channel **44** for receiving cartridge **42.** Channel **44** is open at one end (opening **46**) and closed at the other. Deployed within channel **44** is a loading spring **48** for applying a loading force on a piston **50** of cartridge **42** when inserted within channel **44**. Loading spring **48** is deployed to generate the loading force acting along a direction of extension of channel **44** towards opening **46.** It is a particular feature of this aspect of the present invention that there is provided a retention configuration deployed near opening **46** which includes a plurality of rearwardly directed resilient teeth **52.** Resilient teeth **52** are configured: (a) to retain loading spring **48** in a pre-compressed state within channel **44** prior to insertion of the cartridge (FIG. 5A); (b) to flex outwards to allow insertion of cartridge **42** between teeth **52** (FIG. 5B); and (c) to assume a retaining position engaging a recessed portion of the cartridge when the cartridge is fully inserted (FIG. 5C).

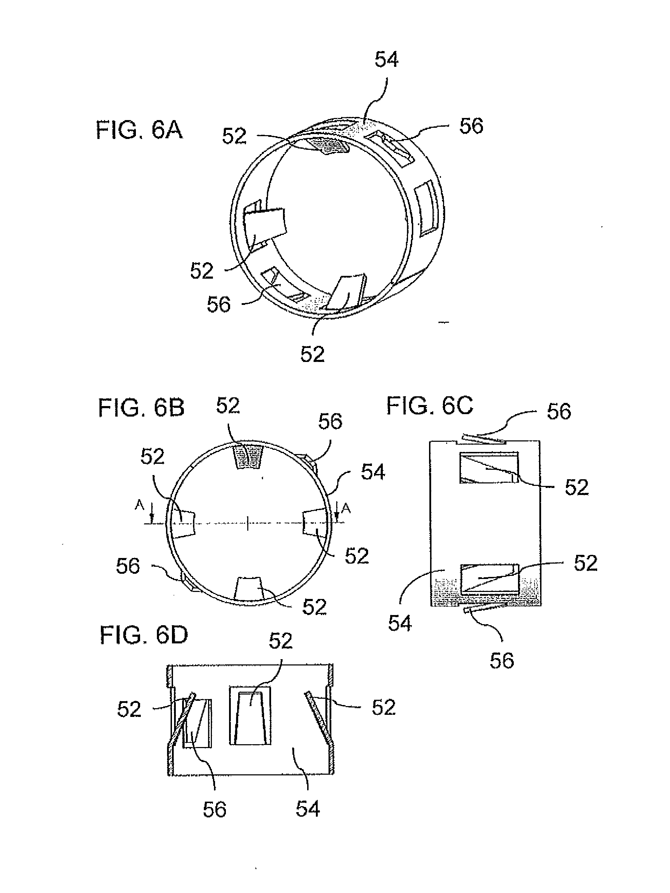

In a particularly preferred implementation illustrated in FIGS. 6A-6D, the plurality of rearward directed resilient teeth **52** are formed as part of a collar **54.** Preferably, at least three, and most preferably at least four, teeth **52** are spaced around the collar, providing symmetrical retention and a centering effect for the head of the cartridge. Teeth **52** are described as "rearwardly directed" in that they project towards the back of channel **44** as viewed from opening **46.** They are also clearly "inwardly projecting", i.e., sloped rearwardly inwards towards the axis of insertion of cartridge **42.** The flexible teeth thus deployed act as unidirectional locking elements, momentarily deforming during insertion of the cartridge and then straightening towards their released position as they reach the narrower recessed portion. An additional advantage of this structure is that the flexible teeth tend to grip cartridge **42** during insertion, even prior to reaching its fully locked position, typically by biting into the layer of paper present in the labeling of the cartridge. This facilitates safe insertion of the cartridge, ensuring that it is not accidentally launched across the room in the event that the user loosens his or her grasp on the cartridge before it is fully inserted.

In the preferred implementation shown here, collar **54** additionally features one or more forward-directed outward projections **56** which serve to lock collar **54** within opening **46.** The entire structure of collar **54** with teeth **52** and projections **56** may be formed from a suitable spring metal, such as stainless steel, by conventional production techniques, such as by stamping, or may be formed from resilient polymer materials, again by conventional production techniques, such as by injection molding.

It will be appreciated that the alignment of cartridge **42** once inserted is defined by forward biasing of the entire cartridge by loading spring **48** into abutment with the ends of teeth **52,** thereby accurately defining the position of the head of the cartridge independent of slight variations in cartridge diameter or length. This facilitates interchangeable use of prefilled cartridges according to the specifications of various different commercially available products. Additionally, according to a further optional feature of the present invention, the drug delivery device may be provided as a kit including an optional user-fillable cartridge sized and shaped for insertion into channel **44,** thereby leaving the user to choose whether to purchase prefilled containers or to fill his or her own container.

The retention arrangement as described is particularly suitable for use in a disposable unit **40** as mentioned, since cartridge **42** is typically not easily removed from the retention arrangement. After use, the cartridge is typically discarded together with disposable unit **40.** In the case illustrated here, drug delivery device is a semi-disposable device in which disposable unit **40** is used together with a reusable control system. However, this and other aspects of the present invention may also be implemented in various other types of device such as, for example, a fully disposable single-use pump.

### User Selectable Infusion Set Form Factor

Turning now to a further aspect of the present invention described with reference to FIGS. 7-10B, this relates to a user selectable form factor for the infusion set to be used with drug delivery device **12,** allowing the user to choose each time the disposable components of the system are switched whether to use an integrated infusion set in a body-mounted device configuration or a separate flexible-tube-fed infusion system for the upcoming round of drug delivery. In each case, all components coming in contact with the drug are preferably single-use disposable components which are switched each time the drug reservoir is replaced, thereby allowing frequent switching or alternating between the two configurations.

Turning now to FIG. 7, this shows a modular drug delivery system kit, generally designated **100,** for delivering a drug to the body of a user. In general terms, kit **100** includes a drug delivery device, such as device **12,** including a disposable unit **40** having an outlet arrangement with at least one, and more preferably two, outlets **102, 104** for release of the drug for infusion into the body of the user. A first infusion set **106** includes a canula **108** in fluid connection with a length of flexible tube **110** for delivery of the drug into the body of the user at a location spaced from drug delivery device **12.** A second infusion set **112** includes a canula **114** deployable in fixed relation to a body contact element **116** which is itself configured to cooperate with drug delivery device **12** to define a fixed spatial alignment between body contact element **116** and drug delivery device **12.** Each infusion set **106** and **112** is configured to be connected to an outlet **102** or **104** of the outlet arrangement to define a flow path from the outlet arrangement to the respective canula **108** or **114** to provide a user selected form factor for the drug delivery system.

Although both infusion sets could be configured for connection to a single outlet, it is a particularly preferred feature of this aspect of the present invention that the outlet arrangement includes at least two separate outlets **102** and **104,** both in fluid connection with the downstream side of the flow control arrangement of the drug delivery device. This allows appropriate choice of positioning and/or orientation for the flow connection for each infusion set. For example, it is typically advantageous to connect external infusion set **106** at an outlet **102** located near a periphery of drug delivery device **12,** while the integrated infusion set **112** is typically advantageously connected at an outlet **104** spaced away from said periphery of drug delivery device **12.** Each outlet is preferably sealed prior to use by a suitable seal arrangement, and the unused outlet remains sealed during the entire operation of the drug delivery.

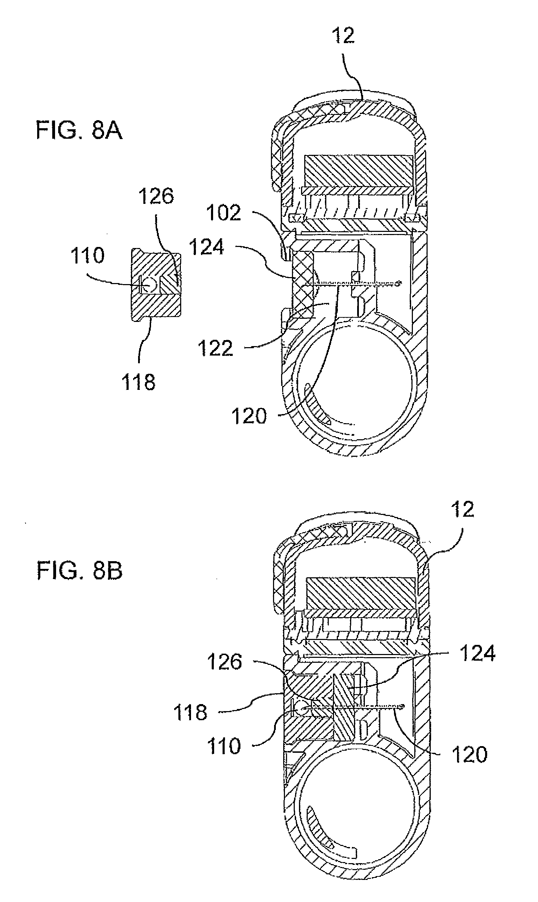

One preferred implementation for the form of outlets **102** and **104,** and the corresponding flow connectors of infusion sets **106** and **112** will be illustrated in the following Figures. Turning first for FIGS. 8A and 8B, these show cross-sectional views through outlet **102** together with a connector **118** of infusion set **106.** As seen here, outlet **102** is formed with a septum needle **120** recessed within a socket **122,** and with an elastomer seal **124** deployed within socket **122** so as to seal the end of septum needle **120** prior to attachment of connector **118** (FIG. 8A). When connector **118** is inserted into socket **122,** elastomer seal **124** and a seal **126** of connector **118** are successively pierced by septum needle **120** until the needle comes into fluid flow connection with flexible tube **110.** Infusion set **106** is then ready for use, with outlet **104** remaining sealed throughout.

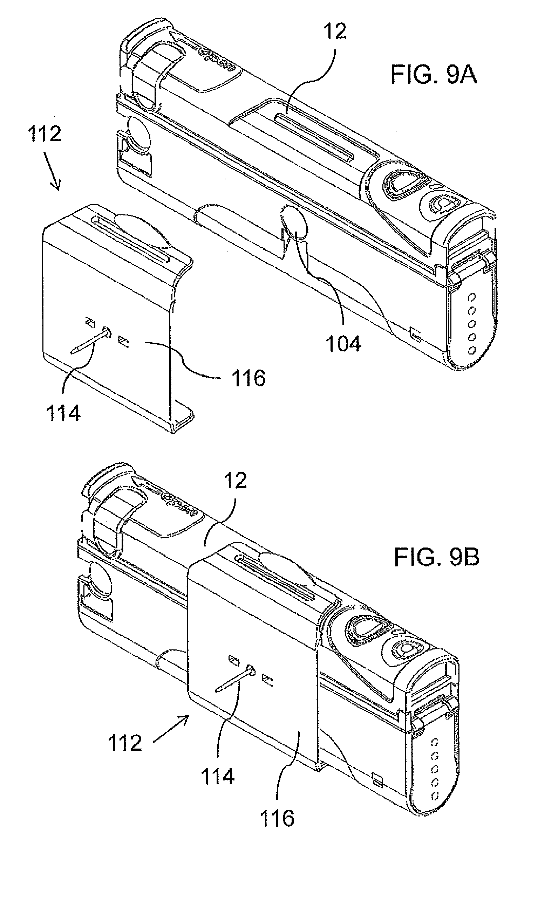

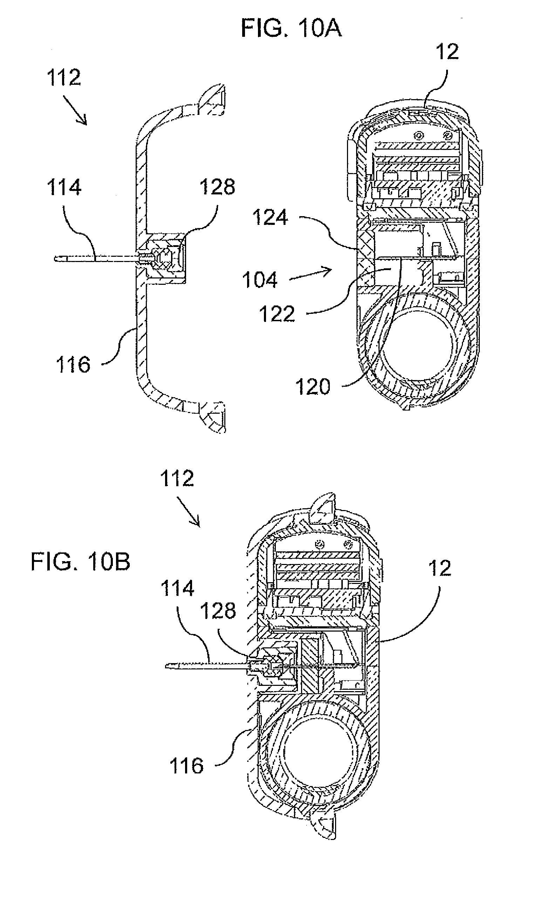

Turning now to Figures 9A-10B, this illustrates the use of integrated infusion set **112.** As best seen in Figure 10A, body contact element **116** is preferably integrated with canula **114** and a connector **128.** Body contact element **116** is configured for attachment to the skin of the user, such as by adhesive or any other conventional form of attachment. Canula **114** is then inserted, typically by use of an insertion mechanism of conventional type, so as to penetrate into the tissue beneath the base. The remaining components of drug delivery device **12** then directly engage body contact element **116** as shown in FIG. 10B, thereby forming a unitary skin-mounted assembly according to the integrated infusion set functionality described above. The mating of connector **128** with outlet **104** occurs in a manner analogous to the mating of connector **118** with outlet **102,** as described above. In this case, outlet **102** remains sealed throughout use of the device in this form factor.

It will be appreciated that the above descriptions are intended only to serve as examples, and that many other embodiments are possible within the scope of the present invention as defined in the appended claims.

## Claims

1. A modular drug delivery system kit for delivering a drug to the body of a user, the kit comprising:
(a) a drug delivery device comprising a disposable unit having an outlet arrangement with at least one outlet for release of a drug for infusion into the body of the user;
(b) a first infusion set including a canula in fluid connection with a length of flexible tube for delivery of the drug into the body of the user at a location spaced from said drug delivery device, said first infusion set being configured for fluid connection to said outlet arrangement; and
(c) a second infusion set including a canula deployable in fixed relation to a body contact element, said body contact element being configured to cooperate with said drug delivery device to define a fixed spatial alignment between said body contact element and said drug delivery device, and to define a fluid flow path from said outlet arrangement to said canula,
wherein one of said first infusion set and said second infusion set is attached to said drug delivery device to provide a user selected form factor for the drug delivery system.

2. The modular drug delivery system kit of claim 1, wherein said outlet arrangement includes a first outlet for attachment of said first infusion set and a second outlet for attachment of said second infusion set.

3. The modular drug delivery system kit of claim 1, wherein said at least one outlet is formed with a septum needle recessed within a socket, and with an elastomer seal deployed within said socket so as to seal the end of said septum needle prior to attachment of said first or second infusion set.
